Europäisches Patentamt

European Patent Office

Office européen des brevets

(11) Veröffentlichungsnummer: **0 012 331**
**A1**

(12) **EUROPÄISCHE PATENTANMELDUNG**

(21) Anmeldenummer: **79104908.3**

(22) Anmeldetag: **04.12.79**

(51) Int. Cl.³: **A 61 B 5/02**

(30) Priorität: **08.12.78 DE 2853098**

(43) Veröffentlichungstag der Anmeldung: **25.06.80**
**Patentblatt 80/13**

(84) Benannte Vertragsstaaten: **AT BE CH DE FR GB IT LU**
**NL SE**

(71) Anmelder: **CLINICON INTERNATIONAL GMBH,**
**Sandhofer Strasse 176, D-6800 Mannheim 31 (DE)**

(72) Erfinder: **Rebbe, Klaus, Bodelschwinghweg 23,**
**D-6800 Mannheim 31 (DE)**
Erfinder: **Rösicke, Bernd, Wormserstrasse 35,**
**D-6800 Mannheim 31 (DE)**
Erfinder: **Wellmann, Klaus, Meerfeldstrasse 46,**
**D-6800 Mannheim 1 (DE)**

(74) Vertreter: **Daum, Martin, Dr. et al, Boehringer Mannheim**
**GmbH. Sandhoferstrasse 116, D-6800 Mannheim 31 (DE)**

(54) **Sphygmomanometer.**

㉝ Die Erfindung betrifft ein Sphygmomanometer mit einer aufblasbaren, mit einer Schnalle (10) versehenen Manschette (11) und einem Schallaufnehmer (20). Die vom Blutdruck abhängigen Signale des Schallaufnehmers werden entweder unmittelbar erkannt und mit der Druckanzeige korreliert oder von einer elektrischen Schaltung verarbeitet und an mindestens eine die Blutdruckwerte wiedergebende Anzeigevorrichtung (17) geliefert. Eine besonders kompakte Bauweise und einfache Handhabung des erfindungsgemässen Sphygmomanometers wird nun dadurch erreicht, dass die Schnalle (10) der Manschette (11) mit dem Gehäuse für die Anzeigevorrichtung (17) und für die elektrische Schaltung zur Umwandlung der Schallsignale eine Einheit bildet.

CLINICON INTERNATIONAL GMBH                    2258 f

Sphygmomanometer

Die Erfindung betrifft ein Sphygmomanometer mit einer aufblasbaren, mit einer Schnalle versehenen Manschette und einem
Schallaufnehmer dessen vom Blutdruck abhängige Signale entweder unmittelbar erkannt und mit der Druckanzeige korreliert
oder von einer elektrischen Schaltung verarbeitet und an
mindestens eine die Blutdruckwerte wiedergebende Anzeigevorrichtung geliefert werden.

Aus der DT-AS 22 20 233 und der US-PS 2,582,123 ist bekannt,
die Meßgeräte direkt an der Manschette anzubringen. Der
Vorteil der etwas kompakteren Bauweise wird mit dem Nachteil
aufgewogen, daß die Meßgeräte während einer Messung von einer
Person, an der die Messung vorgenommen wird, nicht selbst
ablesbar sind.

Der Erfindung lag die Aufgabe zugrunde, Blutdruckmeßgeräte mit
noch kompakterer Bauweise zu schaffen, die obendrein von einer
Person einfach bedient und selbst abgelesen werden können.

Diese Aufgabe wird dadurch gelöst, daß die Schnalle der
Manschette mit dem Gehäuse für die Anzeigevorrichtung und
für die elektrische Schaltung zur Umwandlung der Schallsignale eine Einheit bildet.

Besonders vorteilhaft und kompakt sind solche Sphygmomanometer,
bei denen eine Vorrichtung zum manuellen Aufpumpen der
Manschette in unmittelbarer Nähe der Manschette und der
Befestigungsschnalle unlösbar angebracht ist.

Weitere vorteilhafte Ausgestaltungen sind in den Unteransprüchen
und der nun folgenden Beschreibung der in der Zeichnung schematisch dargestellten Ausführungsbeispiele beschrieben.

Es zeigen:

Figur 1 in perspektivischer Ansicht ein Sphygmomanometer mit Klemmschnalle und Pumpgerät am freien Ende der Manschette.

Figur 2 in perspektivischer Ansicht ein Sphygmomanometer mit an anderer Stelle beschriebener Schnalle mit haftendem Belag.

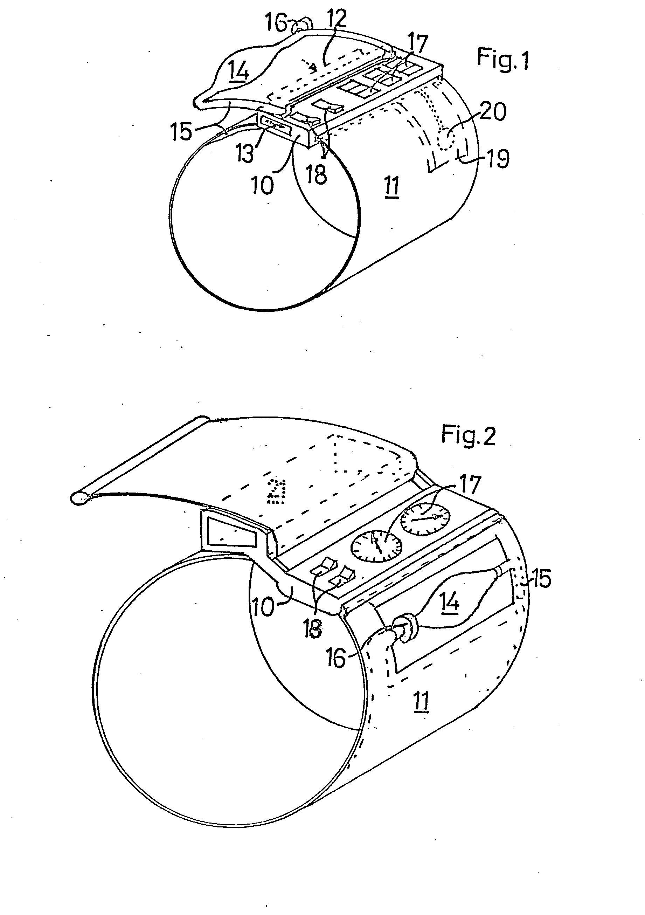

Die in Figur 1 dargestellte Schnalle 10 einer Manschette 11 erstreckt sich über die gesamte Breite des Manschettenbandes. Die Schnalle 10 hat etwa quaderförmige Gestalt. Sie ist mit einer gestrichelt dargestellten Klemmvorrichtung 12 ausgestattet, die von seitlich angebrachten Schiebern 13 auslösbar gestaltet ist.

Figur 1 ist weiterhin zu entnehmen, daß alle Geräteteile zusammengefaßt wurden, indem die Schnalle 10 der Manschette 11 als Gehäuse für die Anzeigvorrichtung 17 ausgebildet ist.

Die sonst notwendige lange und unhandliche Schlauchleitung von der Manschette zum Manometer für die Blutdruckanzeige ist durch eine kurze Durchführung von der Manschette 11 in die Schnalle 10 ersetzt.

Am freien Ende der Manschette ist direkt eine Vorrichtung 14 zum Aufblasen der Manschette über biegsame Rohre 15 angebracht. Es ist erfindungsgemäß nicht mehr nötig, mit einer lose mit der Manschette verbundenen Aufblaspumpe zu arbeiten, weil Pumpe 14, Ventil 16 und Manschette 11 eine Einheit bilden. Dadurch wird das Gerät wesentlich handlicher und kann einfacher bedient werden, weil die störenden Schlauchleitungen nicht mehr vorhanden sind.

Bei elektrischen Sphygmomanometern enthält das Gehäuse außer den Bedienungselementen 18 eine gedruckte Schaltung für die Signalverarbeitung, um automatisch Blutdruckwerte (Systole und Diastole) auf verschiedenen Anzeigeeinheiten anzuzeigen.

- 3 -

Wenn die Energieversorgung der elektrischen Schaltung nicht gleichzeitig durch ein Gerät, beispielsweise einen Dynamo, erfolgt, der beispielsweise mit der Pumpe 14 verbunden ist, dann wird in der Schnalle 10 eine Batterie oder ein Akkumulator untergebracht.

An dem Gehäuse der Schnalle 10 ist außerdem ein Fortsatz 19 angebracht, der unterhalb des Manschettenbandes zu liegen kommt und gestrichelt dargestellt ist. Der Fortsatz 19 ist als Basis für einen Schallaufnehmer 20 vorgesehen. Bei üblicher Befestigungsweise hat sich herausgestellt, daß die Abhörposition des Schallaufnehmers 20 nicht identisch mit der Lage der Schnalle ist.

Auch vereinfacht sich die Fertigung einer solchen Manschette, weil eine Schnalle 10 mit dem Fortsatz 19 einstückig, zum Beispiel aus Spritzgußteilen, herstellbar ist und die gesonderte Montage eines Schallaufnehmers in der Manschette entfällt.

In Figur 2 ist ebenfalls perspektivisch eine andere Version der neuen Manschette dargestellt. Für die gleichen Teile werden die gleichen Bezugszeichen verwendet. Das trifft auch für die Anzeigelemente zu, weil es für die Erfindung ohne Belang ist, ob Rundinstrumente oder Ziffernanzeigen verwendet werden.

Es ergeben sich konstruktive Erleichterungen, wenn die Vorrichtung 14 zum Aufpumpen der Manschette in eine Ausnehmung der Manschette eingelassen ist, da auch bei diesem Ausführungsbeispiel keinerlei lange Schlauchverbindungen notwendig sind und eine direkte Verbindungsleitung zwischen Aufpumpvorrichtung 14 und den Instrumenten 17 in der Schnalle 10 möglich ist.

– 4 –

Die Schnalle 10 der in Figur 2 dargestellten Manschette weist ferner die von der Anmelderin in der Anmeldung P 28 43 643 beschriebene Besonderheit auf, daß ein Belag 21 vorgesehen ist, an dem ein Teil des Manschettenbandes haftet. Die Erfindung eignet sich besonders für solche Manschetten, weil deren Schnallen gegenüber gewöhnlichen Schnallen ohnehin etwas größer ausfallen und leicht die Geräte aufnehmen können, die sie als Gehäuse enthalten sollen.

Bezugszeichen:

10  Schnalle
11  Manschettenband
12  Klemmvorrichtung
13  Betätigungshebel der Klemmvorrichtung
14  Aufblasvorrichtung
15  Luftschlauch
16  Ventil
17  Anzeigegeräte
18  Schalter
19  Fortsatz des Gehäuses
20  Schallaufnehmer
21  haftende Oberfläche der Schnalle (10)

CLINICON INTERNATIONAL GMBH                    2258 f

## Ansprüche

1. Sphygmomanometer mit einer aufblasbaren, mit einer Schnalle versehenen Manschette und einem Schallaufnehmer dessen vom Blutdruck abhängige Signale entweder unmittelbar erkannt und mit der Druckanzeige korreliert oder von einer elektrischen Schaltung verarbeitet und an mindestens eine die Blutdruckwerte wiedergebende Anzeigevorrichtung geliefert werden, dadurch gekennzeichnet, daß die Schnalle (10) der Manschette (11) mit dem Gehäuse für die Anzeigevorrichtung (17) und für die elektrische Schaltung zur Umwandlung der Schallsignale eine Einheit bildet.

2. Sphygmomanometer nach Anspruch 1, dadurch gekennzeichnet, daß eine Vorrichtung (14) zum manuellen Aufpumpen der Manschette (11) in unmittelbarer Nähe der Manschette (11) und der Befestigungsschnalle (10) angebracht ist.

3. Sphygmomanometer nach Anspruch 2, dadurch gekennzeichnet, daß die Vorrichtung (14) zum manuellen Aufpumpen der Manschette (11) am freien Ende des Bandes der Manschette (11) angebracht ist (Fig. 1).

4. Sphygmomanometer nach einem der Ansprüche 2 und 3, dadurch gekennzeichnet, daß die Vorrichtung (14) zum manuellen Aufpumpen der Manschette (11) mit einem Gerät zur Versorgung der Schaltung und der Anzeigevorrichtung mit ausreichender Energie verbunden ist.

5. Sphygmomanometer nach einem der Ansprüche 1 bis 4, dadurch gekennzeichnet, daß die Schnalle (10) ein bewegliches in einer Position feststellbares Teil (12) enthält, mit dem die Manschette (11) auf Zugbelastung befestigbar ist.

6.  Sphygmomanometer nach einem der Ansprüche 1 bis 4, dadurch gekennzeichnet, daß zur vorübergehenden Feststellung der Manschette (11) auf der Schnalle (10) ein Belag (21) angebracht ist, auf dem das freie Ende der Manschette (11) haftet (Fig. 2).

7.  Sphygmomanometer nach einem der vorhergehenden Ansprüche, dadurch gekennzeichnet, daß Anzeigevorrichtungen (17) verwendet werden, die den umgewandelten Meßwert zumindest einige Sekunden lang speichern.

8.  Sphygmomanometer nach einem der vorhergehenden Ansprüche, dadurch gekennzeichnet, daß an der Schnalle (10) und/oder am Gehäuse eine biegsame Haltevorrichtung (19) für mindestens einen Schallaufnehmer (20) starr (10) angebracht ist.

0012331

CLINICON INTERNATIONAL GMBH 2258 f

Fig.1

Fig.2

0012331

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | AT - B - 167 721 (DIBOLD M.) <br> + Gesamt + <br> -- | 1,5,8 |
| | CH - A5 - 598 802 (TAMM U.) <br> + Gesamt + <br> -- | 1,8 |
| | US - A - 2 714 379 (RAINES T.A.) <br> + Gesamt + <br> -- | 1 |
| A | FR - A1 - 2 262 952 (DARRAS I.C.) <br> + Gesamt + <br> -- | 1 |
| A | US - A - 2 841 149 (MARSDEN G.W.) <br> + Gesamt + <br> -- | 2,5 |
| A | DE - C - 566 052 (PLESCH I.) <br> + Gesamt + <br> -- | 5 |
| A | DE - B2- 2 609 339 (BAYER AG) <br> + Gesamt + <br> -- | 7 |
| D | DE - B2 - 2 220 233 (SPEIDEL & KELLER) <br> + Gesamt + <br> -- | 1 |
| D | US - A - 2 582 123 (HEITZ E.) <br> + Gesamt + <br> ---- | 1 |

**KLASSIFIKATION DER ANMELDUNG (Int.Cl.)** 3

A 61 B 5/02

**RECHERCHIERTE SACHGEBIETE (Int.Cl.)** 3

A 61 B 5/00
A 61 B 17/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 13-03-1980 | LUDWIG |

EPA form 1503.1  06.78